(19) Europäisches Patentamt
European Patent Office
Office européen des brevets

(11) Veröffentlichungsnummer: **0 301 338**
**A1**

# (12) EUROPÄISCHE PATENTANMELDUNG

(21) Anmeldenummer: **88111482.1**

(22) Anmeldetag: **16.07.88**

(51) Int. Cl.[4]: **C07D 231/44 , C07D 401/12 , A01N 43/56 , //(C07D401/12, 231:44,213:61)**

(30) Priorität: **28.07.87 DE 3724919**

(43) Veröffentlichungstag der Anmeldung: **01.02.89 Patentblatt 89/05**

(84) Benannte Vertragsstaaten: **AT BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnoeckel 49**
**D-5600 Wuppertal 11(DE)**
Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Becker, Benedikt, Dr.**
**Metzkausener Strasse 14**
**D-4020 Mettmann(DE)**
Erfinder: **Behrenz, Wolfgang, Dr.**
**Untergrundemich 14**
**D-5063 Overath(DE)**
Erfinder: **Homeyer, Bernhard, Dr.**
**Obere Strasse 28**
**D-5090 Leverkusen 3(DE)**
Erfinder: **Stendel, Wilhelm, Dr.**
**In den Birken 55**
**D-5600 Wuppertal 1(DE)**

## (54) 1-Arylpyrazole.

(57) Es werden neue 1-Arylpyrazole der allgemeinen Formel (I)

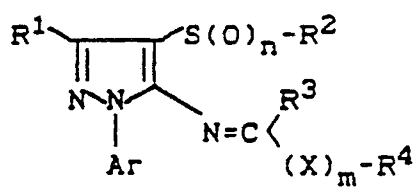

(I)

EP 0 301 338 A1

bereitgestellt, in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$R^3$ für Wasserstoff, Alkyl oder Halogenalkyl steht,
$R^4$ für Alkyl, Alkenyl, Alkinyl, Halogenalkyl, Halogenalkenyl, Halogenalkinyl oder für jeweils gegebenenfalls substituiertes Aryl oder Heteroaryl steht,
X für Sauerstoff, Schwefel oder für einen N-Alkylrest steht,
Ar für jeweils gegebenenfalls substituiertes Phenyl oder Pyridyl steht,
m für eine Zahl 0 oder 1 steht und
n für eine Zahl 0, 1 oder 2 steht.
Die neuen Verbindungen der Formel (I) besitzen eine stark ausgeprägte insektizide Wiksamkeit.

# 1-Arylpyrazole

Die Erfindung betrifft neue 1-Arylpyrazole, mehrere Verfahren zu ihrer Herstellung und ihre Verwendung als Schädlingsbekämpfungsmittel.

Es ist bereits bekannt, daß bestimmte 1-Arylpyrazole wie beispielsweise das 5-(N-Methylamino)-4-trifluormethylthio-1-(2,6-dichlor-4-trifluormethyl-phenyl)-pyrazol oder das 5-(N-Methylamino)-4-dichlorfluormethylthio-1-(2,6-dichlor-4-trifluormethylphenyl)-pyrazol gute insektizide Eigenschaften besitzen (vgl. EP 201 852).

Die Wirksamkeit dieser vorbekannten Verbindungen ist jedoch insbesondere bei niedrigen Aufwandmengen und Konzentrationen nicht bei allen Schadinsekten sowie nicht in allen Anwendungsbereichen völlig zufriedenstellend.

Es wurden neue 1-Arylpyrazole der allgemeinen Formel (I),

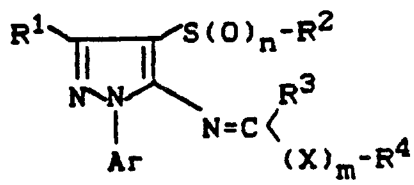

(I)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$R^3$ für Wasserstoff, Alkyl oder Halogenalkyl steht,
$R^4$ für Alkyl, Alkenyl, Alkinyl, Halogenalkyl, Halogenalkenyl, Halogenalkinyl oder für jeweils gegebenenfalls substituiertes Aryl oder Heteroaryl steht,
X für Sauerstoff, Schwefel oder für einen N-Alkylrest steht,
Ar für jeweils gegebenenfalls substituiertes Phenyl oder Pyridyl steht,
m für eine Zahl 0 oder 1 steht und
n für eine Zahl 0, 1 oder 2 steht,
gefunden.

Weiterhin wurde gefunden, daß man die neuen 1-Arylpyrazole der allgemeinen Formel (I),

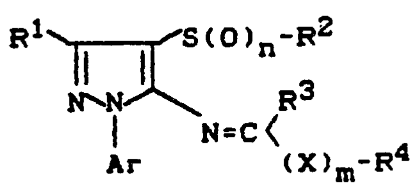

(I)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$R^3$ für Wasserstoff, Alkyl oder Halogenalkyl steht,
$R^4$ für Alkyl, Alkenyl, Alkinyl, Halogenalkyl, Halogenalkenyl, Halogenalkinyl oder für jeweils gegebenenfalls substituiertes Aryl oder Heteroaryl steht,
X für Sauerstoff, Schwefel oder für einen N-Alkylrest steht,
Ar für jeweils gegebenenfalls substituiertes Phenyl oder Pyridyl steht,
m für eine Zahl 0 oder 1 steht und
n für eine Zahl 0, 1 oder 2 steht,
nach einem der im Folgenden beschriebenen Verfahren erhält:

(a) Man erhält 1-Arylpyrazole der Formel (Ia),

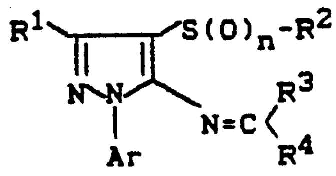

(Ia)

in welcher
$R^1$, $R^2$, $R^3$, $R^4$, Ar und n die oben angegebene Bedeutung haben, wenn man 5-Amino-1-aryl-pyrazole der Formel (II),

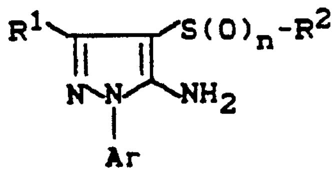

(II)

in welcher
$R^1$, $R^2$, Ar und n die oben angegebene Bedeutung haben, mit Aldehyden oder Ketonen der Formel (III),

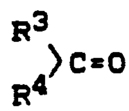

(III)

in welcher
$R^3$ und $R^4$ die oben angegebene Bedeutung haben, gegebenenfalls in Gegenwart eines Verdünnungsmittels und gegebenenfalls in Gegenwart eines Reaktionshilfsmittels umsetzt;

(b) man erhält 1-Arylpyrazole der Formel (Ib),

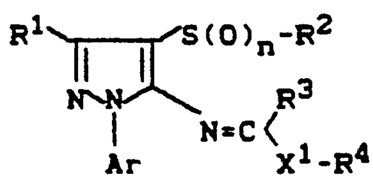

(Ib)

in welcher
$X^1$ für Sauerstoff oder für einen N-Alkylrest steht und
$R^1$, $R^2$, $R^3$, $R^4$, Ar und n die oben angegebene Bedeutung haben, wenn man 5-Amino-1-arylpyrazole der Formel (II),

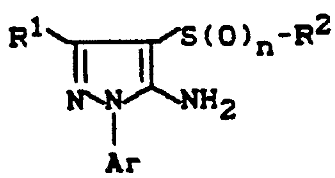

(II)

in welcher
$R^1$, $R^2$, Ar und n die oben angegebene Bedeutung haben, mit Orthoestern oder -amiden der Formel (IV),

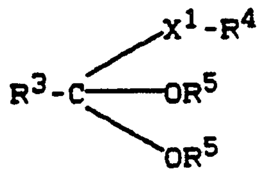

(IV)

in welcher
$R^5$ für Alkyl steht und
$R^3$, $R^4$ und $X^1$ die oben angegebene Bedeutung haben,
gegebenenfalls in Gegenwart eines Verdünnungsmittels und gegebenenfalls in Gegenwart eines Reaktionshilfsmittels umsetzt;

(c) man erhält 1-Arylpyrazole der Formel (Ic),

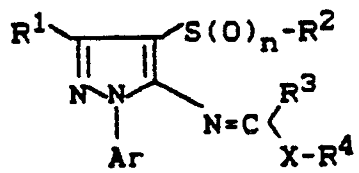

(Ic)

in welcher
$R^1$, $R^2$, $R^3$, $R^4$, X, Ar und n die oben angegebene Bedeutung haben,
wenn man 5-Halogenalkylidenimino-1-aryl-pyrazole der Formel (V),

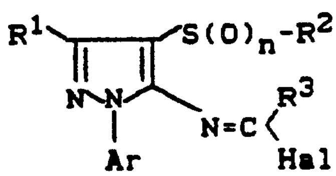

(V)

in welcher
$R^1$, $R^2$, $R^3$, Ar und n die oben angegebene Bedeutung haben und
Hal für Halogen steht,
mit Alkoholen, Aminen oder Thiolen der Formel (VI),

$R^4$-XH (VI)

in welcher
$R^4$ und X die oben angegebene Bedeutung haben,
gegebenenfalls in Gegenwart eines Verdünnungsmittels und gegebenenfalls in Gegenwart eines Reaktionshilfsmittels umsetzt;

(d) man erhält 1-Arylpyrazole der Formel (Id),

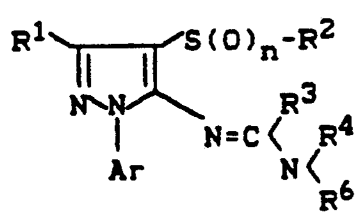

(Id)

in welcher
$R^6$ für Alkyl steht und
$R^1$, $R^2$, $R^3$, $R^4$, Ar und n die oben angegebene Bedeutung haben,
wenn man 1-Arylpyrazole der Formel (Ie),

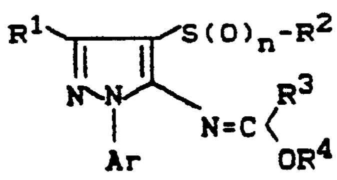

(Ie)

in welcher
$R^1$, $R^2$, $R^3$, $R^4$, Ar und n die oben angegebene Bedeutung haben,
mit Aminen der Formel (VII),

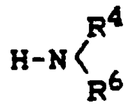

(VII)

in welcher
$R^4$ und $R^6$ die oben angegebene Bedeutung haben,
gegebenenfalls in Gegenwart eines Verdünnungsmittels umsetzt.

Schließlich wurde gefunden, daß die neuen 1-Arylpyrazole der allgemeinen Formel (I) eine gute Wirkung gegen Schädlinge besitzen.

Überraschenderweise zeigen die erfindungsgemäßen 1-Arylpyrazole der allgemeinen Formel (I) eine erheblich bessere insektizide Wirksamkeit, als die aus dem Stand der Technik bekannten 1-Arylpyrazole, wie beispielsweise das 5-(N-Methylamino)-4-trifluormethylthio-1-(2,6-dichlor-4-trifluormethylphenyl)-pyrazol oder das 5-(N-methylamino)-4-dichlorfluormethylthio-1-(2,6-dichlor-4-trifluormethylphenyl)-pyrazol, welche chemisch und wirkungsmäßig naheliegende Verbindungen sind.

Die erfindungsgemäßen 1-Arylpyrazole sind durch die Formel (I) allgemein definiert. Bevorzugt sind Verbindungen der Formel (I), bei welchen

$R^1$ für Wasserstoff oder für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen steht,

$R^2$ für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für Wasserstoff oder für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen steht,

$R^4$ für jeweils geradkettiges oder verzweigtes Alkyl, Alkenyl, Alkinyl mit jeweils bis zu 8 Kohlenstoffatomen, für jeweils geradkettiges oder verzweigtes Halogenalkyl, Halogenalkenyl oder Halogenalkinyl mit jeweils bis zu 8 Kohlenstoffatomen und 1 bis 17 gleichen oder verschiedenen Halogenatomen oder für jeweils gegebenenfalls einfach oder mehrfach, gleich oder verschieden substituiertes Phenyl oder Heteroaryl mit 1 bis 9 Kohlenstoffatomen und 1 bis 3 Heteroatomen, insbesondere Stickstoff, Sauerstoff und/oder Schwefel steht, wobei als Substituenten jeweils die bei $R^2$ genannten Phenylsubstituenten infrage kommen,

X für Sauerstoff, Schwefel oder für einen N-Alkylrest mit 1 bis 6 Kohlenstoffatomen im geradkettigen oder verzweigten Alkylteil steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^7$, wobei

$R^7$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,
m für eine Zahl 0 oder 1 steht,
n für eine Zahl 0, 1 oder 2 steht und
p für eine Zahl 0, 1 oder 2 steht.

Besonders bevorzugt sind Verbindungen der Formel (I), bei welchen
$R^1$ für Wasserstoff, Methyl, Ethyl, n- oder i-Propyl und Trifluormethyl steht,
$R^2$ für Methyl, Ethyl, n- oder i-Propyl, n-, i-, s-oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrom methyl, Fluorchlorbrommethyl oder für jeweils gegebenenfalls einfach bis dreifach, gleich oder verschieden substituiertes Phenyl, Benzyl oder Phenylethyl steht, wobei als Phenylsubstituenten jeweils infrage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,
$R^3$ für Wasserstoff, Methyl, Ethyl, n- oder i-Propyl, n-, i-, s- oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Dichlormethyl, Chlorpropyl, Brompropyl, Chlorfluormethyl, Trichlormethyl, Trifluorethyl, Trifluorchlorethyl, Tetrafluorethyl, Difluorchlorethyl, Fluordibrommethyl, Difluorbrommethyl oder Fluorchlorbrommethyl steht,
$R^4$ für Methyl, Ethyl, n- oder i-Propyl, n-, i-, s-oder t-Butyl, n- oder i-Pentyl, n- oder i-Hexyl, für Vinyl, Propenyl, Allyl, n- oder i-Butenyl, n-oder i-Pentenyl, Propargyl, n- oder i-Butinyl, Chlormethyl, Difluormethyl, Difluorchlormethyl, Fluordichlormethyl, Trifluormethyl, Pentafluor ethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl oder für jeweils gegebenenfalls ein- bis dreifach, gleich oder verschieden substituiertes Phenyl, Pyridyl, Furyl oder Thienyl steht, wobei als Substituenten jeweils die bei $R^2$ genannten Phenylsubstituenten infrage kommen,
X für Sauerstoff, Schwefel oder für einen N-Alkylrest mit 1 bis 4 Kohlenstoffatomen im geradkettigen oder verzweigten Alkylteil steht,
Ar für gegebenenfalls ein- bis fünffach, gleich oder verschiedenen substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^7$, wobei
$R^7$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht,
m für eine Zahl 0 oder 1 steht,
n für eine Zahl 0, 1 oder 2 steht und
p für eine Zahl 0, 1 oder 2 steht.

Ganz besonders bevorzugt sind Verbindungen der Formel (I), bei welchen
$R^1$ für Wasserstoff oder Methyl steht,
$R^2$ für Methyl, Ethyl, Trifluormethyl, Dichlorfluormethyl oder Chlordifluormethyl steht,
$R^3$ für Wasserstoff, Methyl, Ethyl, n- oder i-Propyl oder Trifluormethyl steht,
$R^4$ für Methyl, Ethyl, n- oder i-Propyl, n-, i-, s-oder t-Butyl, Vinyl, Propenyl, Allyl, n- oder i-Butenyl, oder für gegebenenfalls ein- bis dreifach, gleich oder verschieden durch Fluor, Chlor, Brom, Methyl oder Triflurormethyl substituiertes Phenyl steht,
X für Sauerstoff, Schwefel, für einen N-Methylrest oder einen N-Ethylrest steht,
Ar für gegebenenfalls ein- bis fünffach gleich oder verschieden substituiertes Phenyl steht, wobei als Substituenten infrage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl,
m für eine Zahl 0 oder 1 steht und

n für eine Zahl 0, 1 oder 2 steht.

Im einzelnen seien außer den bei den Herstellungsbeispielen genannten Verbindungen die folgenden 1-Arylpyrazole der allgemeinen Formel (I) genannt:

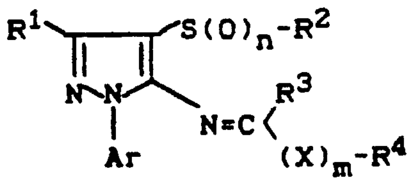

(I)

| $R^1$ | $-S(O)_n-R^2$ | $-N=C\langle^{R^3}_{(X)_mR^4}$ | Ar |
|---|---|---|---|
| H | $-SO_2CF_3$ | 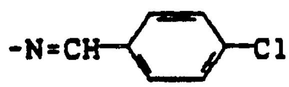 | 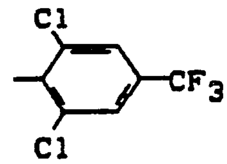 |
| H | $-SOCF_3$ | 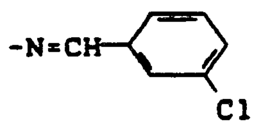 | 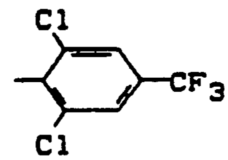 |
| H | $-SO_2CF_3$ | 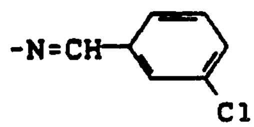 | 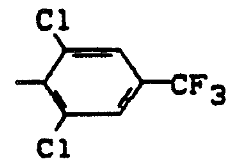 |
| H | $-SOCF_3$ | 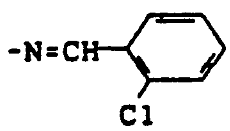 | 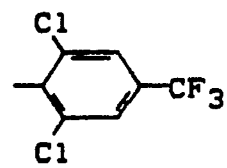 |
| H | $-SO_2CF_3$ | 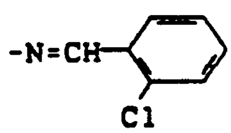 | 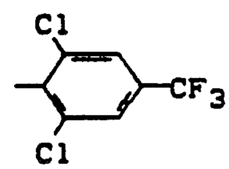 |
| H | $-SOCCl_2F$ | 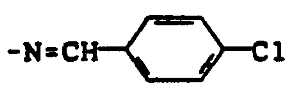 | 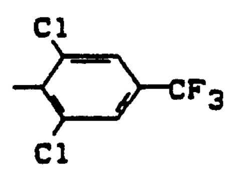 |

| $R^1$ | $-S(O)_n-R^2$ | $-N{=}C\langle {R^3 \atop (X)_mR^4}$ | Ar |
|---|---|---|---|
| H | $-SO_2CCl_2F$ | 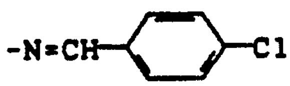 | 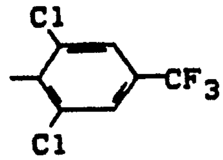 |
| H | $-SOCCl_2F$ | 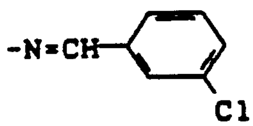 | 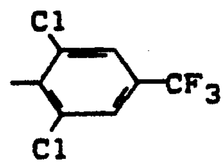 |
| H | $-SCF_3$ | 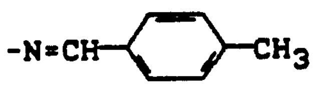 | 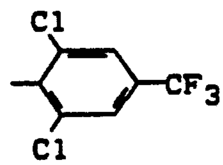 |
| H | $-SO_2CF_3$ | 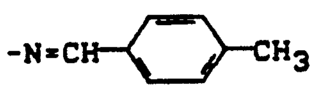 | 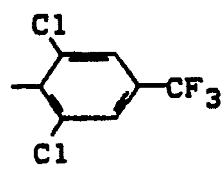 |
| H | $-SCF_3$ | 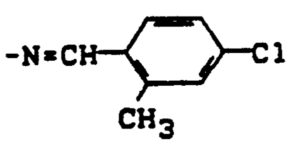 | 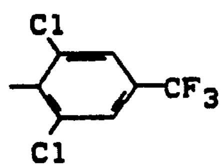 |

Verwendet man beispielsweise 5-Amino-1-(2,6-dichlor-4-trifluormethylphenyl)-4-trifluormethylthio-pyrazol und Benzaldehyd als Ausgangsstoffe, so läßt sich der Reaktionsablauf des erfindungsgemäßen Verfahrens (a) durch das folgende Formelschema darstellen:

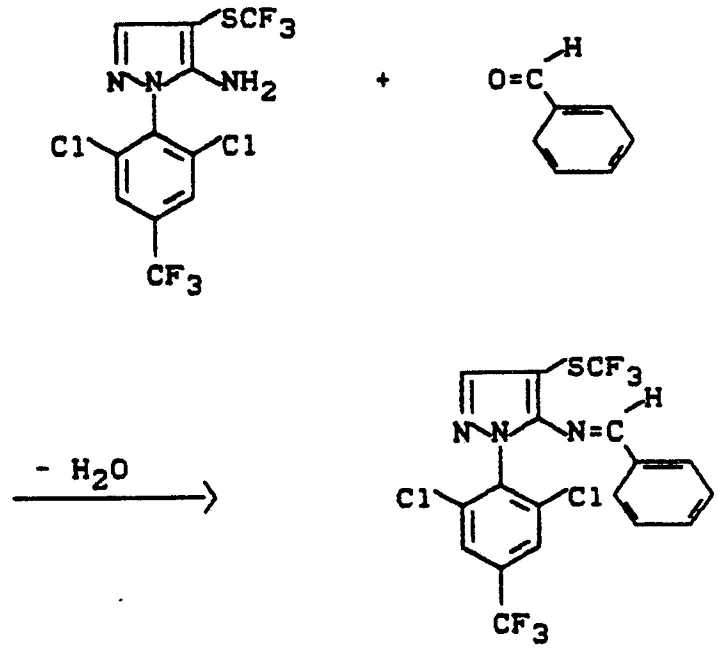


Verwendet man beispielsweise 5-Amino-4-dichlorfluormethylthio-1-(2-fluor-6-chlor-4-trifluormethylphenyl)pyrazol und Ortho-Ameisensäuretriethylester als Ausgangsstoffe, so läßt sich der Reaktionsablauf des erfindungsgemäßen Verfahrens (b) durch das folgende Formelschema darstellen:

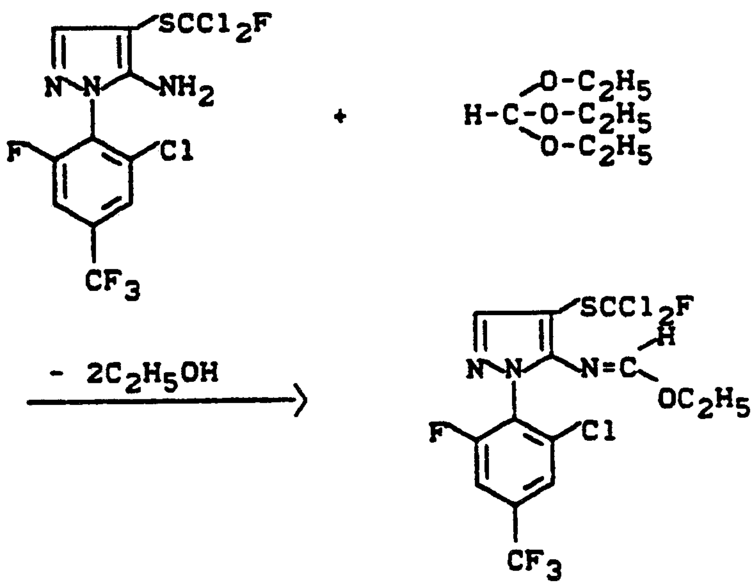


Verwendet man beispielsweise 5-(α-Chlorpropylidenimino)-4-difluorchlormethylsulfonyl-1-(2,6-dichlor-4-brom-phenyl)-pyrazol und n-Butylmercaptan als Ausgangsstoffe, so läßt sich der Reaktionsablauf des erfindungsgemäßen Verfahrens (c) durch das folgende Formelschema darstellen:

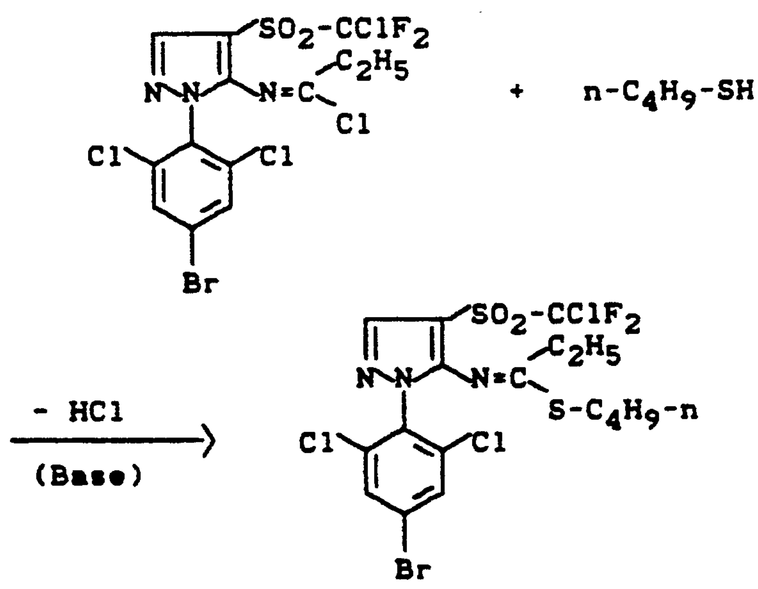


Verwendet man beispielsweise 5-Methoxymethyliden-imino-3-methyl-4-trifluormethylsulfinyl-1-(4-trifluormethylphenyl)-pyrazol und Diethylamin als Ausgangsstoffe, so läßt sich der Reaktionsablauf des erfindungsgemäßen Verfahrens (d) durch das folgende Formelschema darstellen:

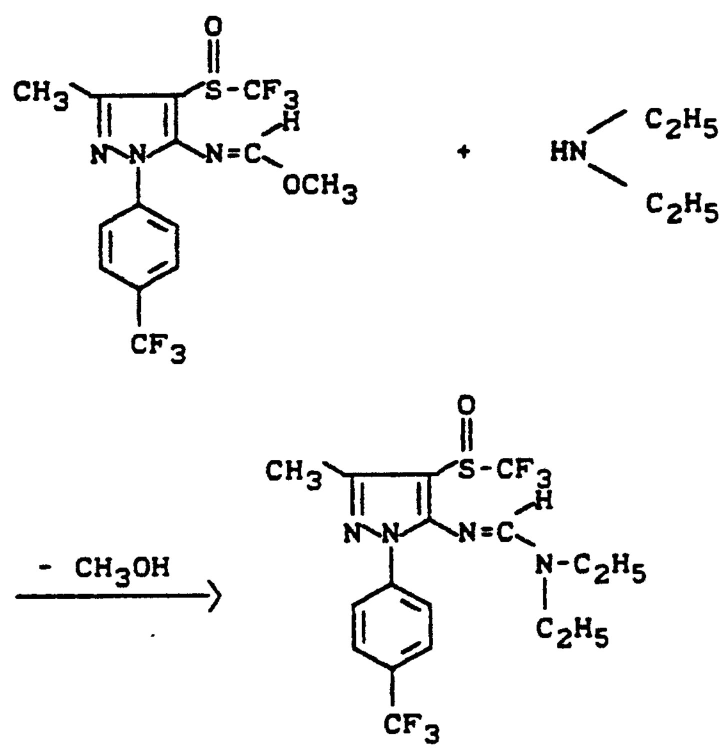


Die zur Durchführung der erfindungsgemäßen Verfahren (a) und (b) als Ausgangsstoffe benötigten 5-Amino-1-aryl-pyrazole sind durch die Formel (II) allgemein definiert. In dieser Formel (II) stehen $R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind bekannt oder lassen sich in Analogie zu bekannten Verfahren erhalten (vgl. EP 201 852).

Die zur Durchführung des erfindungsgemäßen Verfahrens (a) weiterhin als Ausgangsstoffe benötigten Aldehyde oder Ketone sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aldehyde oder Ketone der Formel (III) sind allgemein bekannte Verbindungen der organischen Chemie.

Die zur Durchführung des erfindungsgemäßen Verfahrens (b) weiterhin als Ausgangsstoffe benötigten Orthoester und -amide sind durch die Formel (IV) allgemein definiert. In dieser Formel (IV) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

$X^1$ steht vorzugsweise für Sauerstoff oder für einen geradkettigen oder verzweigten N-Alkylrest mit 1 bis 6, insbesondere mit 1 bis 4 Kohlenstoffatomen.

$R^5$ steht vorzugsweise für geradkettiges oder verzweigtes Alkyl mit 1 bis 4 Kohlenstoffatomen, insbesondere für Methyl oder Ethyl.

Die Orthoester und -amide der Formel (IV) sind allgemein bekannte Verbindungen der organischen Chemie.

Die zur Durchführung des erfindungsgemäßen Verfahrens (c) als Ausgangsstoffe benötigten 5-Halogenalkyliden-imino-1-arylpyrazole sind durch die Formel (V) allgemein definiert. In dieser Formel (V) stehen $R^1$, $R^2$, $R^3$, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Hal steht vorzugsweise für Chlor oder Brom.

Die 5-Halogenalkylidenimino-1-aryl-pyrazole der Formel (V) sind noch nicht bekannt. Man erhält sie, wenn man 5-Acylamino-1-aryl-pyrazole der Formel (VIII),

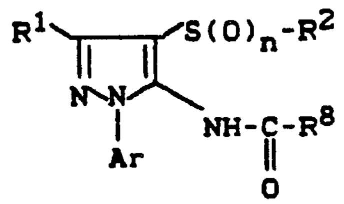

(VIII)

in welcher

$R^1$, $R^2$, Ar und n die oben angegebene Bedeutung haben und

$R^8$ für Alkyl, insbesondere für Methyl oder Ethyl steht,

mit üblichen Halogenierungsmitteln, wie beispielsweise Phosphorpentachlorid oder Phosphortribromid gegebenenfalls in Gegenwart eines Verdünnungsmittels wie beispielsweise Dichlormethan bei Temperaturen zwischen 20 °C und 120 ° umsetzt.

Die 5-Acylamino-1-arylpyrazole der Formel (VIII) sind bekannt oder lassen sich in Analogie zu bekannten Verfahren erhalten (vgl. EP 201 852).

Die zur Durchführung des erfindungsgemäßen Verfahrens (c) weiterhin als Ausgangsstoffe benötigten Alkohole, Amine oder Thiole sind durch die Formel (VI) allgemein definiert. In dieser Formel (VI) stehen $R^4$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die Alkohole, Amine und Thiole der Formel (VI) sind allgemein bekannte Verbindungen der organischen Chemie.

Die zur Durchführung des erfindungsgemäßen Verfahrens (d) als Ausgangsstoffe benötigten 1-Arylpyrazole sind durch die Formel (Ie) allgemein definiert. In dieser Formel (Ie) stehen $R^1$, $R^2$, $R^3$, $R^4$, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Aryl-pyrazole der Formel (Ie) sind erfindungsgemäße Verbindungen und erhältlich mit Hilfe der erfindungsgemäßen Verfahren (b) oder (c).

Die zur Durchführung des erfindungsgemäßen Verfahrens (d) weiterhin als Ausgangsstoffe benötigten Amine sind durch die Formel (VII) allgemein definiert. In dieser Formel (VII) steht $R^4$ vorzugsweise für diejenigen Reste, die bereits im Zusammenhang mit der Beschreibung der erfindungsgemäßen Stoffe der Formel (I) als bevorzugt für diesen Substituenten genannt wurden.

$R^6$ steht vorzugsweise für geradkettiges oder verzweigtes Alkyl mit 1 bis 6 Kohlenstoffatomen, besonders bevorzugt für geradkettiges oder verzweigtes Alkyl mit 1 bis 4 Kohlenstoffatomen, insbesondere für Methyl oder Ethyl.

Die Amine der Formel (VII) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere alipha tische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Nitrile, wie Acetonitril oder Propionitril, Ester, wie Essigsäureethylester oder Sulfoxide, wie Dimethylsulfoxid.

Das erfindungsgemäße Verfahren (a) wird vorzugsweise in Gegenwart eines geeigneten Reaktionshilfsmittels durchgeführt.

Als solche eignen sich Säuren, wie beispielsweise Schwefelsäure oder Chlorwasserstoffsäure, Basen wie beispielsweise Natriumhydroxid, Kaliumhydroxid oder Triethylamin oder wasserentziehende Mittel wie beispielsweise Natriumsulfat oder Molekularsieb.

Es ist auch möglich, freiwerdendes Reaktionswasser durch azeotrope Destillation aus dem Reaktionsgemisch zu entfernen.

Die Reaktionstemperaturen können bei der Durchführung des erfindungsgemäßen Verfahrens (a) in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens (a) setzt man pro Mol an 5-Amino-1-aryl-pyrazole der Formel (II) im allgemeinen 1.0 bis 10.0 Mol, vorzugsweise 1.0 bis 5.0 Mol an Aldehyd oder Keton der Formel (III) und gegebenenfalls 0.01 bis 5.0 Mol, vorzugsweise 0.1 bis 1.5 Mol an Reaktionshilfsmittel ein.

Die Reaktionsdurchführung, Aufarbeitung und Isolierung der Reatkionsprodukte erfolgt nach allgemein üblichen Methoden.

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Nitrile, wie Acetonitril oder Propionitril oder Alkohole, wie Methanol, Ethanol oder Propanol.

Es ist jedoch auch möglich, einen entsprechenden Überschuß an als Reaktionspartner eingesetztem Orthoester oder -amid der Formel (IV) gleichzeitig als Verdünnungsmittel zu verwenden.

Der erfindungsgemäße Verfahren (b) wird gegebenenfalls in Gegenwart eines geeigneten Reaktionshilfsmittels durchgeführt. Als solche kommen insbesondere anorganische oder organische Säuren, wie Salzsäure, Essigsäure oder p-Toluolsulfonsäure infrage. Es ist jedoch auch möglich, das erfindungsgmäße Verfahren (b) ohne Anwesenheit eines Reakionshilfsmittel durchzuführen.

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens (b) setzt man pro Mol an 5-Amino-1-aryl-pyrazol der Formel (II) im allgemeinen 1.0 bis 20.0 Mol, vorzugsweise 1.0 bis 10.0 Mol an Orthoester oder -amid der Formel (IV) und gegebenenfalls 0.01 bis 10.0 Mol, vorzugsweise 0.1 bis 1.5 Mol an Reaktionshilfsmittel ein.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder atomatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Nitrile, wie Acetonitril oder Propionitril, Ester, wie Essigsäureethylester oder Sulfoxide, wie Dimethylsulfoxid.

Es ist auch möglich, einen entsprechenden Überschuß an als Reaktionspartner eingesetztem Alkohol, Amin oder Thiol der Formel (VI) gleichzeitig als Verdünnungsmittel zu verwenden.

Das erfindungsgemäße Verfahren (c) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N.N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen - 20 °C

und + 150 °C, vorzugsweise bei Temperaturen zwischen 0 °C und 120 °C.

Zur Durchführung des erfindungsgemäßen Verfahrens (c) setzt man pro Mol an 5-Halogenalkylidenimino-1-aryl-pyrazol der Formel (V) im allgemeinen 1.0 bis 20.0 Mol, vorzugsweise 1.0 bis 10.0 Mol an Alkohol, Amin oder Thiol der Formel (VI) und gegebenenfalls 1.0 bis 5.0 Mol, vorzugsweise 1.0 bis 2.0 Mol an Reaktionshilfsmit tel ein. Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Nitrile, wie Acetonitril oder Propionitril, Ester, wie Essigsäureethylester oder Sulfoxide, wie Dimethylsulfoxid.

Es ist jedoch auch möglich einen entsprechenden Überschuß an als Reaktionspartner eingesetztem Amin der Formel (VII) gleichzeitig als Verdünnungsmittel zu verwenden.

Die Reaktionstemperaturen können bei der Durchführung des erfindungsgemäßen Verfahrens (d) in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens (d) setzt man pro Mol an 1-Arylpyrazol der Formel (Ie) im allgemeinen 1.0 bis 20.0 Mol, vorzugsweise 1.0 bis 5.0 Mol an Amin der Formel (VII) ein. Die Reaktionsdurchführung, Aufarbeitung und Isolierung der Reaktionsprodukte erfolgt nach allgemein üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda, z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodop tera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Os cinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken. Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe insektizide Wirksamkeit aus. Sie lassen sich mit gutem Erfolg gegen pflanzenschädigende Insekten, wie beispielsweise gegen die Larven der Meerrettichblattkäfer (Phaedon cochleariae), gegen die grüne Pfirsichblattlaus (Myzus persicae) oder gegen die schwarze Bohnenblattlaus (Aphis fabae) einsetzen. Dabei zeigen die erfindungsgemäßen Wirkstoffe nicht nur protektive sondern auch blattsystemische und wurzelsystemische Eigenschaften.

Daneben eignen sich die erfindungsgemäßen Wirkstoffe auch zur Bekämpfung von Bodeninsekten und lassen sich beispielsweise zur Bekämpfung der Maden der Zwiebelfliege (Phorbia antiqua) im Boden einsetzen.

Außerdem besitzen die erfindungsgemäßen Wirkstoffe eine hohe Wirkung gegen Hygiene- und Vorratsschädlinge und lassen sich beispielsweise zur Bekämpfung der deutschen Schabe (Blattella germanica) einsetzen.

Darüberhinaus lassen sich die erfindungsgemäßen Wirkstoffe mit besonders gutem Erfolg zur Bekämpfung von parasitisch lebenden Warmblüterschädlingen, wie beispielsweise gegen die Rinderzecke (Boophilus microplus) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure, Aluminiumoxid und Silikate; als feste Träge˙ stoffe für Granulate kommen in Frage: z.B. gebrochene und fraktionierte natürliche Gesteine wie Calcit, Marmor, Bims, Sepiolith, Dolomit sowie synthetische Granulate au anorganischen und organischen Mehlen sowie Granulate aus organischem Material wie Sägemehl, Kokosnußschalen, Maiskolben und Tabakstengel; als Emulgier und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synt hetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stofee u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden:

<u>Herstellungsbeispiele</u>

Beispiel 1

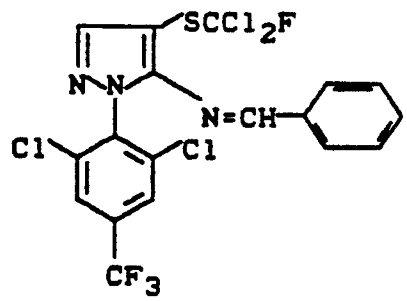

(Verfahren a)

8,6 g (0,02 Mol) 5-Amino-1-(2,6-dichlor-4-trifluormethylphenyl)-4-dichlorfluormethylthio-pyrazol in 200 ml wasserfreiem Toluol werden 4 Stunden über einen Wasserabscheider unter Rückfluß erhitzt. Dann gibt man 4 Tropfen konzentrierte Schwefelsäure zu und tropft innerhalb von 2 Stunden 6,4 g (0,06 Mol) frisch destillierten Benzaldehyd zu. Nach beendeter Zugabe erhitzt man für weitere 16 Stunden auf Rückflußtemperatur und scheidet dabei freiwerdendes Reaktionswasser über einen Wasserabscheider ab. Zur Aufarbeitung wird die abgekühlte Reaktionsmischung dreimal mit jeweils 100 ml gesättigter Natriumhydrogencarbonatlösung gewaschen, über Natriumsulfat getrocknet und im Vakuum eingeengt. Der Rückstand wird säulenchromatographisch (Kieselgel; Laufmittel: Petrolether/Essigester 9 : 1) gereinigt.

Man erhält 8 g (77 % der Theorie) an 5-Benzylidenimino-1-(2,6-dichlor-4-trifluormethyl-phenyl)-4-dichlorfluormethylthio-pyrazol vom Schmelzpunkt 75 °C.

Beispiel 2

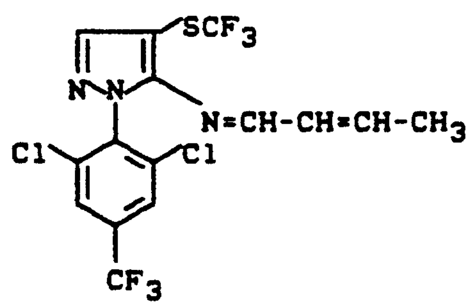

(Verfahren a)

7,9 g (0.02 Mol) 5-Amino-1-(2,6-dichlor-4-trifluormethylphenyl)-4-trifluormethylthio-pyrazol 2,2 g (0.05 Mol) Acetaldehyd, 0.1 g p-Toluolsulfonsäure und 5 g Molekularsieb (BAYLITH TE 144) in 100 ml Toluol werden 48 Stunden bei Raumtemperatur gerührt, anschließend filtriert, das Filtrat im Vakuum eingeengt und der Rückstand säulenchromatographisch gereinigt (Kieselgel; Laufmittel: Petrolether/Essigester).

Man erhält 2,5 g (28 % der Theorie) an 5-But-2-en-1-ylidenimino-1-(2,6-dichlor-4-trifluormethyl-phenyl)-4-trifluormethylthio-pyrazol als Öl.

$^1$H-NMR ($CDCl_3$) : $\delta$ = 1,95 (d, 3H); 7,9 (s, 1H) ppm.

MS: m/e = 447; 449 ($M^+$)

Beispiel 3

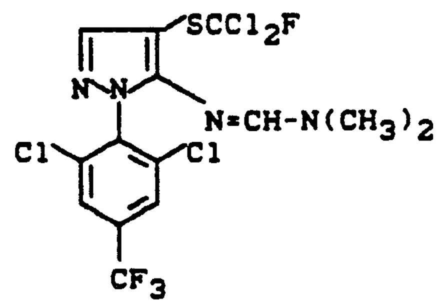

**(Verfahren b)**

10 g (0.025 Mol) 5-Amino-4-dichlorfluormethylthio-1-(2,6-dichlor-4-trifluormethyl-phenyl)-pyrazol in 25 ml (0,23 Mol) Dimethylformamid-dimethylacetal werden 12 Stunden unter Rückfluß erhitzt, anschließend im Vakuum eingeengt, der Rückstand mit Wasser verührt, abgesaugt und getrocknet.

Man erhält 11 g (97 % der Theorie) an 5-(N,N-Dimethylaminomethylidenimino)-1-(2,6-dichlor-4-trifluormethyl-phenyl)-4-dichlorfluormethylthio-pyrazol vom Schmelzpunkt 108 °C (Zers.).

Beispiel 4

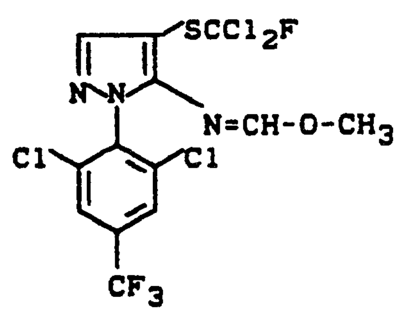

**(Verfahren b)**

13.0 g (0.03 Mol) 5-Amino-1-(2,6-dichlor-4-trifluormethyl-phenyl)-4-dichlorfluormethylthio-pyrazol und 40 ml (0,39 Mol) Orthoameisensäuretrimethylester werden mit 0,5 g p-Toluolsulfonsäure versetzt und 4 Stunden auf Rückflußtemperatur erhitzt. Gleichzeitig destilliert man über eine 10 cm lange Vigreux-Kolonne mit absteigenden Kühler freiwerdendes Methanol aus der Reaktionsmischung ab. Zur Aufarbeitung engt man im Vakuum ein, löst den Rückstand in Ligroin, filtriert über Kieselgur und entfernt das Lösungsmittel im Vakuum.

Man erhält 11,5 g (81 % der Theorie) an 5-(Methoxymethylidenimino)-1-(2,6-dichlor-4-trifluormethyl-phenyl)-4-dichlorfluormethylthio-pyrazol als Öl.

$^1$H-NMR ($CDCl_3$) : $\delta$ = 3,66 (s, 3H); 7,69 (s, 2H); 7,87 (s, 1H); 8,42 (s, 1H) ppm.

In entsprechender Weise und gemäß den allgemeinen Angaben zur Herstellung erhält man die folgenden 1-Arylpyrazole der allgemeinen Formel (I):

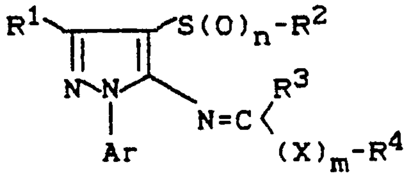

(I)

| Bsp. Nr. | $R^1$ | $-S(O)_n-R^2$ | $=C\langle R^3, (X)_m-R^4$ | Ar | physikal. Daten |
|---|---|---|---|---|---|
| 5 | H | $-SCCl_2F$ | $=CH-N(CH_3)_2$ | 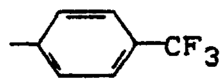 | Fp 77-78 °C |
| 6 | H | $-SCCl_2F$ | $=CH-N(CH_3)_2$ | 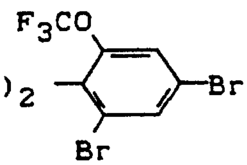 | Fp 92 °C |
| 7 | H | $-SCF_3$ | =CH 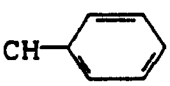 | 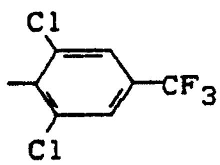 | Fp 85 °C |
| 8 | H | $-SCF_3$ | $=CH-OC_2H_5$ | 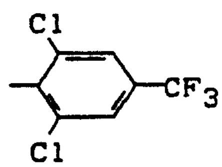 | $^1$H-NMR*) 7,9; 8,35 |

| Bsp. Nr. | $R^1$ | $-S(O)_n-R^2$ | $=C\langle^{R^3}_{(X)_m-R^4}$ | Ar | physikal. Daten |
|---|---|---|---|---|---|
| 9 | H | $-SCCl_2F$ | =CH 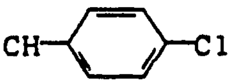 | 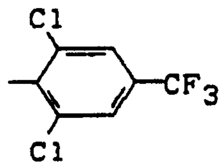 | Fp 95 °C |
| 10 | H | $-SCCl_2F$ | =CH 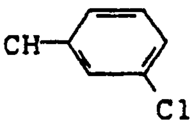 | 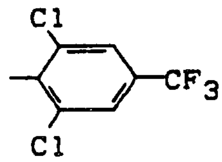 | Fp 91 °C |
| 11 | H | $-SCCl_2F$ | =CH 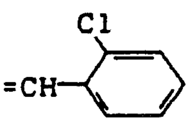 | 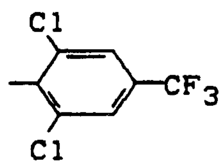 | Fp 105° C |
| 12 | $CH_3$ | $-SCF_3$ | =CH 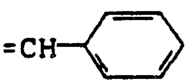 | 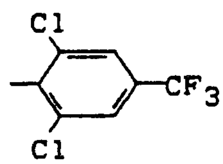 | $^1$H-NMR*) 9,05 |
| 13 | H | 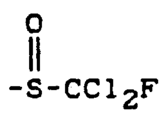 | =CH 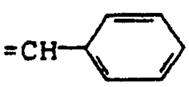 | 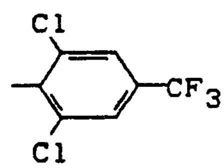 | Fp 110° C |
| 14 | H | $-SO_2-CCl_2F$ | =CH 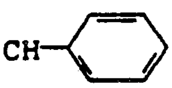 | 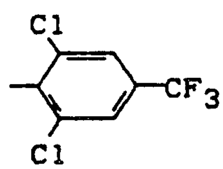 | Fp 159° C |

| Bsp. Nr. | $R^1$ | $-S(O)_n-R^2$ | $=C\langle^{R^3}_{(X)_m-R^4}$ | Ar | physikal. Daten |
|---|---|---|---|---|---|
| 15 | H | $-SO_2-CF_3$ | 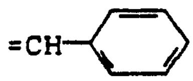 | 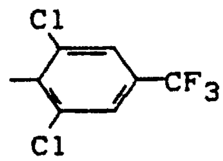 | Fp 132° C |
| 16 | H | 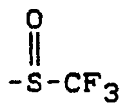 | 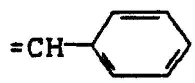 | 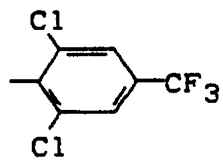 | Fp 141° C |
| 17 | H | 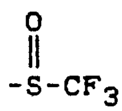 | 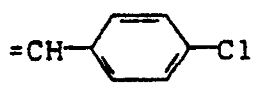 | 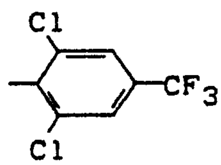 | Fp 118° C |
| 18 | $CH_3$ | $-SCF_3$ | $=CH-OC_2H_5$ | 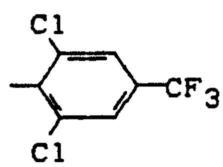 | Fp 39-41 °C |
| 19 | $CH_3$ | $-SCCl_2F$ | $=CH-OC_2H_5$ | 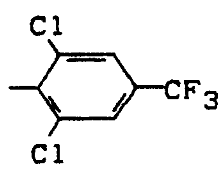 | Fp 76-77 °C |
| 20 | $CH_3$ | $-SO_2-CCl_2F$ | $=CH-OC_2H_5$ | 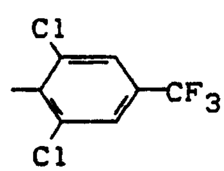 | Fp 111-112° C |
| 21 | $CH_3$ | 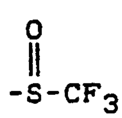 | $=CH-OC_2H_5$ | 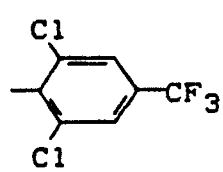 | $n_D^{20}$ 1.5162 |

| Bsp. Nr. | $R^1$ | $-S(O)_n-R^2$ | $=C\langle^{R^3}_{(X)_m-R^4}$ | Ar | physikal. Daten |
|---|---|---|---|---|---|
| 22 | $CH_3$ | $-SO_2-CF_3$ | $=CH-OC_2H_5$ | 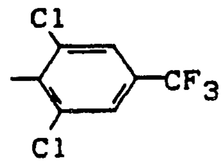 | Fp 67-68 °C |
| 23 | $CH_3$ | $-SCF_3$ | $=C\langle^{CH_3}_{OCH_3}$ | 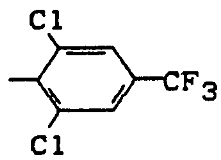 | $n_D^{20}$ 1.5069 |
| 24 | $CH_3$ | $-SCF_3$ | $=C\langle^{C_2H_5}_{OC_2H_5}$ | 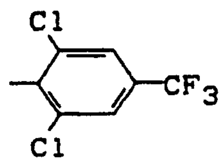 | $n_D^{20}$ 1.5064 |
| 25 | $CH_3$ | $-SCF_3$ | $=CH-OCH_3$ | 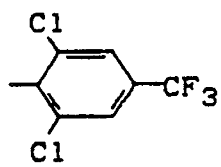 | Fp. 80-81° C |
| 26 | $CH_3$ | $-SCF_3$ | $=C\langle^{CH_3}_{OC_2H_5}$ | 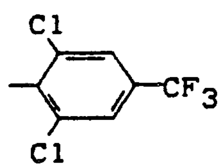 | $n_D^{20}$ 1.520 |
| 27 | H | $-\overset{O}{\overset{\|}{S}}-CF_3$ | =CH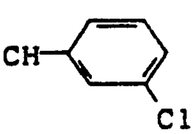 | 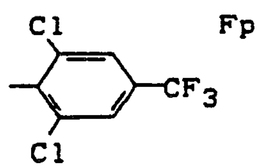 | Fp. 108° C |

| Bsp. Nr. | $R^1$ | $-S(O)_n-R^2$ | $=C\langle^{R^3}_{(X)_m-R^4}$ | Ar | Schmelzpunkt in °C |
|---|---|---|---|---|---|
| 28 | H | $-\overset{O}{\overset{\|}{S}}-CCl_2F$ | =CH 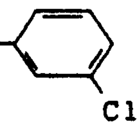 | 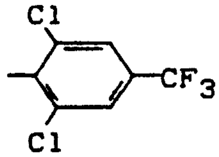 | 110 |
| 29 | $CH_3$ | $-SCCl_2F$ | =CH 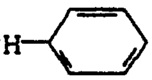 | 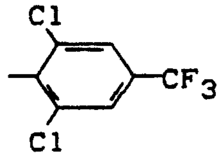 | 127 |
| 30 | $CH_3$ | $-SCF_3$ | =CH 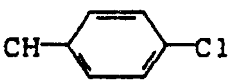 | 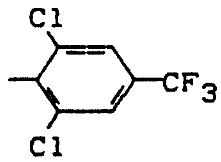 | 104 |
| 31 | $CH_3$ | $-SCCl_2F$ | =CH 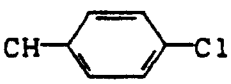 | 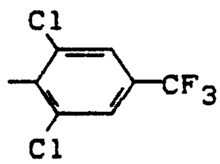 | 125 |
| 32 | $CH_3$ | $-SCF_3$ | =CH 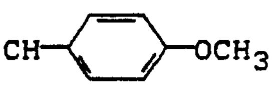 | 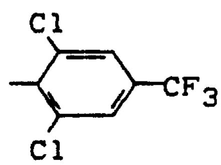 | 130-135 |
| 33 | $CH_3$ | $-SCCl_2F$ | =CH 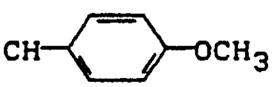 | 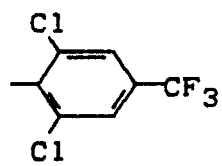 | 97-99 |

| Bsp. Nr. | $R^1$ | $-S(O)_n-R^2$ | $=C\langle^{R^3}_{(X)_m-R^4}$ | Ar | Schmelzpunkt in °C |
|---|---|---|---|---|---|
| 34 | $CH_3$ | $-SCF_3$ | 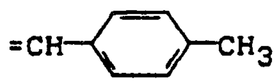 | 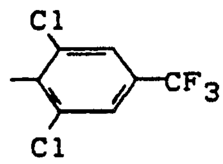 | 120-123 |
| 35 | $CH_3$ | $-SCCl_2F$ | 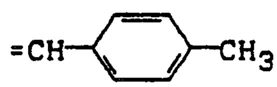 | 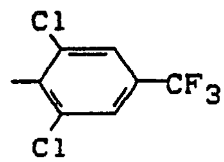 | 122-125 |
| 36 | $CH_3$ | $-SCF_3$ | 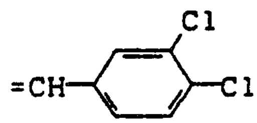 | 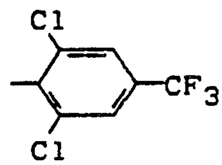 | 94 |
| 37 | $CH_3$ | $-SCCl_2F$ | 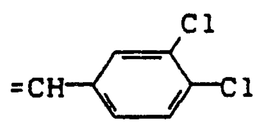 | 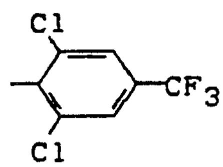 | 91 |
| 38 | $CH_3$ | $-SCF_3$ | 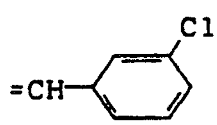 | 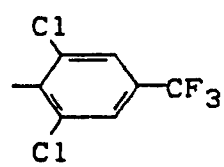 | 110-112 |
| 39 | $CH_3$ | $-SCCl_2F$ | 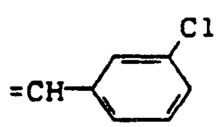 | 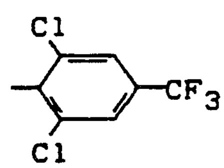 | 59-62 |

| Bsp. Nr. | $R^1$ | $-S(O)_n-R^2$ | $R^3$<br>$=C\langle$<br>$(X)_m-R^4$ | Ar | Schmelzpunkt in °C |
|---|---|---|---|---|---|
| 40 | $CH_3$ | $-SCF_3$ | =CH 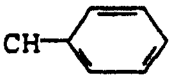 | 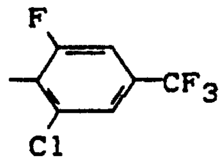 | 83-84 |
| 41 | $CH_3$ | 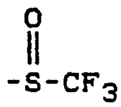 | =CH 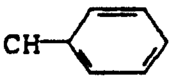 | 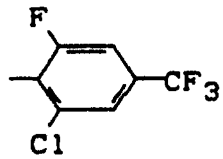 | 89-90 |
| 42 | H | $-SCCl_2F$ | =CH 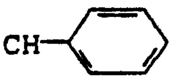 | 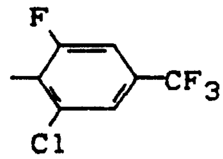 | 103-109 |
| 43 | H | 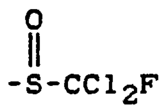 | =CH 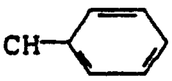 | 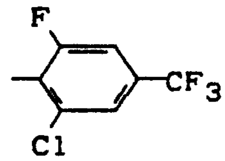 | 88-94 |
| 44 | H | $-SO_2-CCl_2F$ | =CH 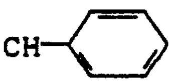 | 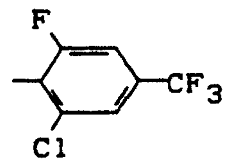 | 116-122 |

*) Die $^{1}H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

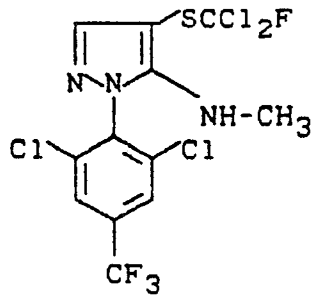

(A)

5-(N-Methylamino)-1-(2,6-dichlor-4-trifluormethyl-phenyl)-4-dichlorfluormethylthio-pyrazol

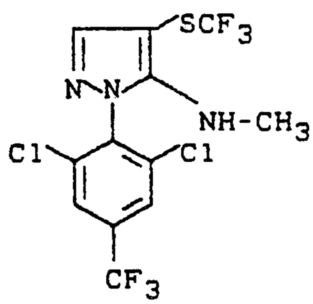

(B)

5-(N-Methylamino)-1-(2,6-dichlor-4-trifluormethyl-phenyl)-4-trifluormethylthio-pyrazol

(beide bekannt aus EP 201 852)

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die

Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigt z.B. die folgende Verbindung der Herstellungsbeispiele überlegene Wirksamkeit gegenüber dem Stand der Technik: 10, 17, 28, 41, 43, 44.

Beispiel B

Aphis-Test (systemische Wirkung)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 2 und 8.

Beispiel C

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigt z.B. die folgende Verbindung der Herstellungsbeispiele überlegene Wirksamkeit gegenüber dem Stand der Technik: 1.

Beispiel D

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzel systemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 7 und 8.

Beispiel E

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweise des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigt z.B. die folgende Verbindung der Herstellungsbeispiele überlegene Wirkung gegenüber dem Stand der Technik: 8.

Beispiel F

$LD_{100}$-Test

Testtiere: Blattella germanica
Zahl der Testtiere: 10
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigt z.B. die folgende Verbindung der Herstellungsbeispiele überlegene Wirkung gegenüber dem Stand der Technik: 2.

Beispiel G

Test mit Boophilus microplus resistent/OP-resistenter Biarra-Stamm

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigt z.B. die folgende Verbindung der Herstellungsbeispiele überlegene Wirkung gegenüber dem Stand der Technik: 4.

**Ansprüche**

1. 1-Arylpyrazole der allgemeinen Formel (I),

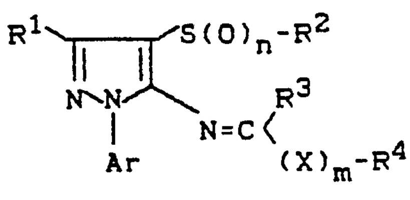

(I)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$R^3$ für Wasserstoff, Alkyl oder Halogenalkyl steht,
$R^4$ für Alkyl, Alkenyl, Alkinyl, Halogenalkyl, Halogenalkenyl, Halogenalkinyl oder für jeweils gegebenenfalls substituiertes Aryl oder Heteroaryl steht,
X für Sauerstoff, Schwefel oder für einen N-Alkylrest steht,
Ar für jeweils gegebenenfalls substituiertes Phenyl oder Pyridyl steht,

m für eine Zahl 0 oder 1 steht und
n für eine Zahl 0, 1 oder 2 steht.

2. 1-Arylpyrazole der allgemeinen Formel (I) gemäß Anspruch 1), in welcher

$R^1$ für Wasserstoff oder für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen steht,

$R^2$ für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlen stoffatomen in den einzelnen Alkylteilen und gegebenenfalls 1 bis 9 gleichen oder verschiedenen Halogenatomen,

$R^3$ für Wasserstoff oder für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen steht,

$R^4$ für jeweils geradkettiges oder verzweigtes Alkyl, Alkenyl, Alkinyl mit jeweils bis zu 8 Kohlenstoffatomen, für jeweils geradkettiges oder verzweigtes Halogenalkyl, Halogenalkenyl oder Halogenalkinyl mit jeweils bis zu 8 Kohlenstoffatomen und 1 bis 17 gleichen oder verschiedenen Halogenatomen oder für jeweils gegebenenfalls einfach oder mehrfach, gleich oder verschieden substituiertes Phenyl oder Heteroaryl mit 1 bis 9 Kohlenstoffatomen und 1 bis 3 Heteroatomen, insbesondere Stickstoff, Sauerstoff und/oder Schwefel steht, wobei als Substituenten jeweils die bei $R^2$ genannten Phenylsubstituenten infrage kommen,

X für Sauerstoff, Schwefel oder für einen Alkylrest mit 1 bis 6 Kohlenstoffatomen im geradkettigen oder verzweigten Alkylteil steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^7$, wobei

$R^7$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

m für eine Zahl 0 oder 1 steht,
n für eine Zahl 0, 1 oder 2 steht und
p für eine Zahl 0, 1 oder 2 steht.

3. 1-Arylpyrazole der allgemeinen Formel (I) gemäß Anspruch 1), in welcher

$R^1$ für Wasserstoff, Methyl, Ethyl, n- oder i-Propyl und Trifluormethyl steht,

$R^2$ für Methyl, Ethyl, n- oder i-Propyl, n-, i-, s-oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl oder für jeweils gegebenenfalls einfach bis dreifach, gleich oder verschieden substituiertes Phenyl, Benzyl oder Phenylethyl steht, wobei als Phenylsubstituenten jeweils infrage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Wasserstoff, Methyl, Ethyl, n- oder i-Propyl, n-, i-, s- oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Dichlormethyl, Chlorpropyl, Brompropyl, Chlorfluormethyl, Trichlormethyl, Trifluorethyl, Trifluorchlorethyl, Tetrafluorethyl, Difluorchlorethyl, Fluordibrommethyl, Difluorbrommethyl oder Fluorchlorbrommethyl steht,

$R^4$ für Methyl, Ethyl, n- oder i-Propyl, n-, i-, s-oder t-Butyl, n- oder i-Pentyl, n- oder i-Hexyl, für Vinyl, Propenyl, Allyl, n- oder i-Butenyl, n-oder i-Pentenyl, Propargyl, n- oder i-Butinyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Dichlorbutenyl, Difluorallyl, Bromallyl oder für jeweils gegebenenfalls ein- bis dreifach, gleich oder verschieden substituiertes Phenyl, Pyridyl, Furyl oder Thienyl steht, wobei als Substituenten jeweils die bei $R^2$ genannten Phenylsubstituenten infrage kommen,

X für Sauerstoff, Schwefel oder für einen N-Alkylrest mit 1 bis 4 Kohlenstoffatomen im geradkettigen oder verzweigten Alkylteil steht,

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluor ethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^7$, wobei

$R^7$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht,

m für eine Zahl 0 oder 1 steht,

n für Zahl 0, 1 oder 2 steht und

p für eine Zahl 0, 1 oder 2 steht.

4. 1-Arylpyrazole der allgemeinen Formel (I) gemäß Anspruch 1),

in welcher

$R^1$ für Wasserstoff oder Methyl steht,

$R^2$ für Methyl, Ethyl, Trifluormethyl, Dichlorfluormethyl oder Chlordifluormethyl steht,

$R^3$ für Wasserstoff, Methyl, Ethyl, n- oder i-Propyl oder Trifluormethyl steht,

$R^4$ für Methyl, Ethyl, n- oder i-Propyl, n-, i-, s- oder t-Butyl, Vinyl, Propenyl, Allyl, n-oder i-Butenyl, oder für gegebenenfalls ein-bis dreifach, gleich oder verschieden durch Fluor, Chlor, Brom, Methyl oder Trifluormethyl substituiertes Phenyl steht,

X für Sauerstoff, Schwefel, für einen N-Methylrest oder einen N-Ethylrest steht,

Ar für gegebenenfalls ein- bis fünffach gleich oder verschieden substituiertes Phenyl steht, wobei als Substituenten infrage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl,

m für eine Zahl 0 oder 1 steht und

n für eine Zahl 0, 1 oder 2 steht.

5. Verfahren zur Herstellung von 1-Arylpyrazolen der allgemeinen Formel (I),

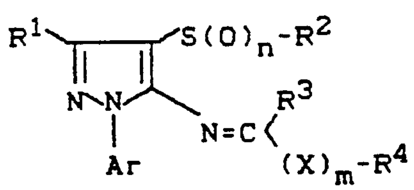

(I)

in welcher

$R^1$ für Wasserstoff, Alkyl oder Halogenalkyl steht,

$R^2$ für Alkyl, Halogenalkyl, für gegebenenfalls substituiertes Aralkyl oder für gegebenenfalls substituiertes Aryl steht,

$R^3$ für Wasserstoff, Alkyl oder Halogenalkyl steht,

$R^4$ für Alkyl, Alkenyl, Alkinyl, Halogenalkyl, Halogenalkenyl, Halogenalkinyl oder für jeweils gegebenenfalls substituiertes Aryl oder Heteroaryl steht,

X für Sauerstoff, Schwefel oder für einen N-Alkylrest steht,

Ar für jeweils gegebenenfalls substituiertes Phenyl oder Pyridyl steht,

m für eine Zahl 0 oder 1 steht und

n für eine Zahl 0, 1 oder 2 steht,

dadurch gekennzeichnet,

(a) daß man zum Erhalt von 1-Arylpyrazolen der Formel (Ia),

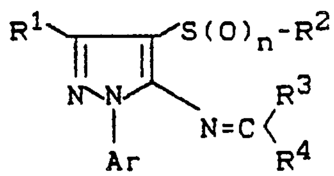

(Ia)

in welcher
$R^1$, $R^2$, $R^3$, $R^4$, Ar und n die oben angegebene Bedeutung haben,
5-Amino-1-aryl-pyrazole der Formel (II),

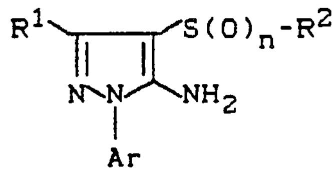

(II)

in welcher
$R^1$, $R^2$, Ar und n die oben angegebene Bedeutung haben,
mit Aldehyden oder Ketonen der Formel (III),

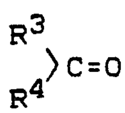

(III)

in welcher
$R^3$ und $R^4$ die oben angegebene Bedeutung haben,
gegebenenfalls in Gegenwart eines Verdünnungsmittels und gegebenenfalls in Gegenwart eines Reaktionshilfsmittels umsetzt;

(b) oder daß man zum Erhalt von 1-Arylpyrazole der Formel (Ib),

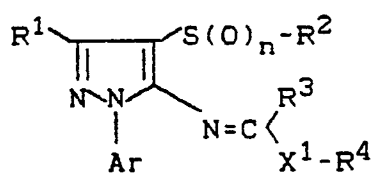

(Ib)

in welcher
$X^1$ für Sauerstoff oder für einen N-Alkylrest steht und
$R^1$, $R^2$, $R^3$, $R^4$, Ar und n die oben angegebene Bedeutung haben,
5-Amino-1-arylpyrazole der Formel (II),

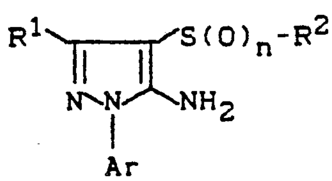

(II)

in welcher
$R^1$, $R^2$, Ar und n die oben angegebene Bedeutung haben,
mit Orthoestern oder -amiden der Formel (IV),

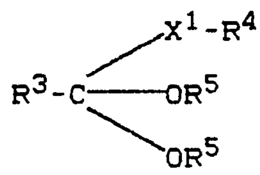

(IV)

in welcher
$R^5$ für Alkyl steht und
$R^3$, $R^4$ und $X^1$ die oben angegebene Bedeutung haben,
gegebenenfalls in Gegenwart eines Verdünnungsmittels und gegebenenfalls in Gegenwart eines Reaktionshilfsmittels umsetzt;

(c) oder daß man zum Erhalt von 1-Arylpyrazolen der Formel (Ic),

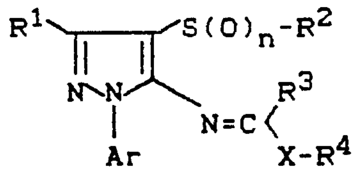

(Ic)

in welcher
$R^1$, $R^2$, $R^3$, $R^4$, X, Ar und n die oben angegebene Bedeutung haben,
5-Halogenalkylidenimino-1-aryl-pyrazole der Formel (V),

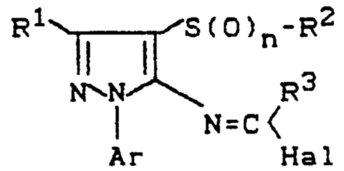

(V)

in welcher
$R^1$, $R^2$, $R^3$, Ar und n die oben angegebene Bedeutung haben und
Hal für Halogen steht,
mit Alkoholen, Aminen oder Thiolen der Formel (VI),

$R^4$-XH (VI)

in welcher
$R^4$ und X die oben angegebene Bedeutung haben,
gegebenenfalls in Gegenwart eines Verdünnungsmittels und gegebenenfalls in Gegenwart eines Reaktionshilfsmittels umsetzt;

(d) oder daß man zum Erhalt von 1-Arylpyrazolen der Formel (Id),

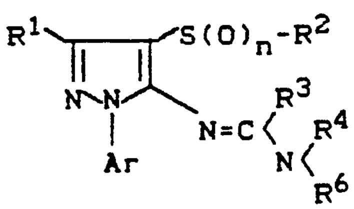

(Id)

in welcher
$R^6$ für Alkyl steht und
$R^1$, $R^2$, $R^3$, $R^4$, Ar und n die oben angegebene Bedeutung haben,
1-Arylpyrazole der Formel (Ie),

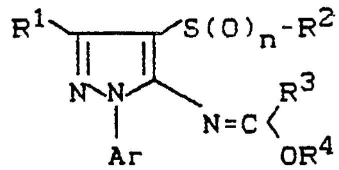

(Ie)

in welcher
$R^1$, $R^2$, $R^3$, $R^4$, Ar und n die oben angegebene Bedeutung haben,
mit Aminen der Formel (VII),

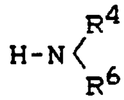

(VII)

in welcher
$R^4$ und $R^6$ die oben angegebene Bedeutung haben,
gegebenenfalls in Gegenwart eines Verdünnungsmittels umsetzt.

6. Schädlingsbekämpfungsmittel, gekennzeichnet durch einen Gehalt an mindestens einem 1-Arylpyrazol der Formel (I).

7. Insektizide Mittel, gekennzeichnet, durch einen Gehalt an mindestens einem 1-Arylpyrazol der Formel (I)

8. Verfahren zur Bekämpfung von tierischen Schädlingen, insbesondere von Insekten, dadurch gekennzeichnet, daß man 1-Arylpyrazole der Formel (I) auf tierische Schädlinge und/oder ihren Lebensraum einwirken läßt.

9. Verwendung von 1-Arylpyrazolen der Formel (I) zur Bekämpfung von tierischen Schädlingen, insbesondere von Insekten.

10. Verfahren zur Herstellung von Mitteln gegen tierische Schädlinge, dadurch gekennzeichnet, daß man 1-Arylpyrazole der Formel (I) mit Streckmitteln und/oder oberflächenaktiven Mitteln vermischt.

11. 5-Halongenalkylidenimino-1-arylpyrazole der Formel (V),

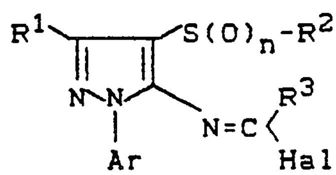

(V)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes Aryl steht,
$R^3$ für Wasserstoff, Alkyl oder Halogenalkyl steht,
Ar für jeweils gegebenenfalls substituiertes Phenyl oder Pyridyl steht,
Hal für Halogen steht und
n für eine Zahl 0, 1 oder 2 steht.

12. Verfahren zur Herstellung von 5-Halogenalkylidenimino-1-aryl-pyrazolen der Formel (V),

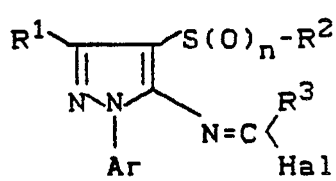

(V)

in welcher
$R^1$ für Wasserstoff, Alkyl oder Halogenalkyl steht,
$R^2$ für Alkyl, Halogenalkyl, für gegebenenfalls substituiertes Aralkyl oder für gegebenenfalls substituiertes

Aryl steht,
$R^3$ für Wasserstoff, Alkyl oder Halogenalkyl steht,
Ar für jeweils gegebenenfalls substituiertes Phenyl oder Pyridyl steht,
Hal für Halogen steht und
n für eine Zahl 0, 1 oder 2 steht.
dadurch gekennzeichnet, daß man 5-Acylamino-1-arylpyrazole der Formel (VIII),

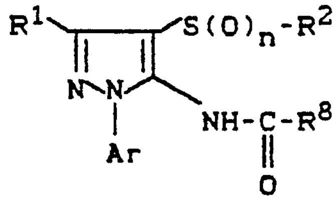

(VIII)

in welcher
$R^1$, $R^2$, Ar und n die oben angegebene Bedeutung haben und
$R^8$ für Alkyl steht,
mit Halogenierungsmitteln, gegebenenfalls in Gegenwart eines Verdünnungsmittels bei Temperaturen zwischen 20° C und 120° umsetzt.

Europäisches Patentamt

# EUROPÄISCHER RECHERCHENBERICHT

Nummer der Anmeldung

EP 88111482.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | DE - A1 - 3 606 476 (BAYER AG)<br>* Ansprüche *<br>-- | 1,5-12 | C 07 D 231/44<br>C 07 D 401/12<br>A 01 N 43/56<br>//(C 07 D 401/12<br>C 07 D 231:44<br>C 07 D 213:61) |
| A | WO - A1 - 87/03 781 (MAY & BAKER LIMITED)<br>* Ansprüche 1,5,10,12 *<br>-- | 1,5-12 | |
| A | CHEMICAL ABSTRACTS, Band 99, Nr. 11, 12. September 1983, Columbus, Ohio, USA<br>GIORI, P. et al. "Synthesis and antifungal activity of pyrazole derivatives containing sulfurated functions"<br>Seite 562, Spalte 1, Zusammenfassung-Nr. 88 101m<br>& Farmaco, Ed. Sci. 1983, 38(4), 274-82<br>-- | 1,5-12 | **RECHERCHIERTE SACHGEBIETE (Int. Cl.4)** |
| A | CHEMICAL ABSTRACTS, Band 75, Nr. 5, 2. August 1971, Columbus, Ohio, USA<br>GIORI, PAOLO et al. "Synthesis and antifungal properties of pyrazolyl mono- and disulfides. II."<br>Seite 498, Spalte 2, Zusammenfassung-Nr. 35 870j<br>& Farmaco, Ed. Sci. 1971, 26(4), 276-93<br>---- | 1,5-12 | C 07 D 231/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 04-11-1988 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82